# DEMANDE DE BREVET EUROPEEN

(11) **EP 2 156 799 A2**
(43) Date de publication de la demande: **24.02.2010**
(21) Numéro de dépôt: 09002928.1
(22) Date de dépôt: 02.07.2003
(51) Int. Cl.: A61B 17/86

(54) **Vis d 'osteosynthese et de compression auto-taraudante et auto-forante**

(30) Priorité: 05.07.2002 FR 0208589
(62) Demande divisionnaire de: 03356101.0
(71) Demandeur: Newdeal S.A.S, 69006 Lyon (FR)
(72) Inventeur: Fourcault, Eric Stéphane, 69004 Lyon (FR); Giet, Jean-Christophe, Alain, 69006 Lyon (FR); Knevels, Theo, Jan. Maria, 1850 Grimbergen (BE); Gauneau, Bertrand, Xavier, François, 69006 Lyon (FR)
(74) Mandataire: Martin, Didier Roland Valéry

(57) **Abrégé**

- Vis d'ostéosynthèse et de compression auto-taraudante et auto-forante.
- L'invention concerne une vis d'ostéosynthèse et de compression formée par un corps longitudinal comprenant :
- une partie proximale (2) formée par une tête de vis (3) pourvue d'un filetage (4), ladite partie proximale (2) étant d'un diamètre supérieur à celui du reste de la vis,
- une partie intermédiaire (5) dépourvue de filetage,
- une partie distale (6) pourvue d'un filetage (7),
- une paire de gorges (10) présentes au niveau de la partie distale (6) et de la partie proximale (2),

la gorge (10) présente au niveau de la partie distale (6) étant de forme hélicoïdale, la zone terminale (8) de la partie distale (6) étant pourvue de moyens de préparation (11) dans les fragments d'os d'un logement destiné à recevoir les parties intermédiaire (5) et distale (6), comprenant au moins une dent (11A).
- Vis d'ostéosynthèse à compression.

## Description

La présente invention se rapporte au domaine technique des vis chirurgicales, en particulier aux vis d'ostéosynthèse destinées à assurer la solidarisation et la compression de deux fragments d'os en vue de réaliser une ostéosynthèse rapide avec formation d'un cal osseux, la présente invention s'appliquant plus particulièrement à la solidarisation de petits fragments d'os tels que ceux des phalanges d'orteils ou de doigts.

La présente invention concerne une vis d'ostéosynthèse et de compression destinée à la coaptation de petits fragments d'os, formée par un corps longitudinal unique comprenant :
- une partie proximale formée par une tête de vis pourvue d'un filetage externe, ladite partie proximale étant d'un diamètre supérieur à celui du reste de la vis,
- une partie intermédiaire dépourvue de filetage,
- une partie distale pourvue d'un filetage externe.

Lors de la rupture de fragments d'os de taille réduite, tels que ceux de phalanges ou d'orteils, la petite dimension des os ou fragments d'os concernée pose des problèmes délicats à résoudre au praticien chargé de réduire la fracture puis de remettre en place les os avec suffisamment de compression pour assurer une bonne résorption de la fracture.

En effet, on sait que pour assurer une ostéosynthèse rapide des deux fragments d'os entre eux, se concrétisant par la formation rapide d'un cal osseux de bonne qualité afin de permettre un retour rapide à une fonction normale, la mise en place et fixation relative des deux petits fragments d'os doit être réalisée avec une compression longitudinale relative des deux fragments d'os.

Il est bien évident que compte-tenu de la très faible dimension des fragments d'os impliqués, et corrélativement des vis d'ostéosynthèse utilisées, la mise en compression longitudinale des deux fragments d'os concernés est difficile à réaliser.

La maîtrise de ce type d'intervention chirurgicale est de plus particulièrement importante et délicate, dans la mesure où les manipulations imposées par la petite taille des os et des vis sont difficiles, alors que le positionnement relatif des petits fragments d'os entre eux ainsi que leur compression et mise en place définitive doivent être réalisés avec une grande précision s'agissant, dans le cas d'os de la main et du pied en particulier, de restaurer la totalité des fonctions de mobilité, telles que les fonctions de manipulation ou de marche.

C'est ainsi que l'on connaît déjà l'utilisation d'agrafes mises en place directement sur les deux fragments d'os à solidariser. Une telle technique est assez mal adaptée au type d'intervention chirurgicale considérée, dans la mesure où la mise en place des agrafes relativement aux parties osseuses ne permet pas d'assurer une position parfaite des fragments d'os à solidariser. De plus, il est pratiquement impossible d'obtenir une fixation en compression avec des agrafes.

C'est ainsi qu'il a déjà été proposé d'utiliser des vis d'ostéosynthèse du genre de celles utilisées pour la coaptation d'os de dimension importante et qui soient à même d'assurer, en plus de la solidarisation des os, une fonction supplémentaire de mise en compression longitudinale.

Ainsi, il a déjà été proposé d'utiliser une vis d'ostéosynthèse et de compression comprenant une partie proximale formée par une tête de vis pourvue d'un filetage externe, et présentant un diamètre supérieur au reste de la vis. Cette vis présente une partie intermédiaire dépourvue de filetage afin d'assurer un glissement relatif amélioré des fragments d'os à solidariser lors du vissage de la vis, ladite partie intermédiaire se poursuivant par une partie distale pourvue elle aussi d'un filetage externe.

De telles vis améliorent grandement les conditions d'intervention chirurgicale, en raison de l'amélioration des facilités de mise en place qu'elles procurent.

Néanmoins, de telles vis souffrent toujours d'inconvénients liés en particulier à un certain nombre d'opérations supplémentaires imposées au chirurgien telles que la réalisation de forage préalable pour assurer une bonne tenue mécanique des filets de la vis à la fois pour la partie distale et pour la partie proximale dont le diamètre de tête est supérieur. Ceci multiplie donc les opérations imposées au chirurgien et augmente la durée d'intervention.

Des améliorations notables ont été apportées à ce type de vis mais elles ont essentiellement porté sur l'augmentation des capacités de compression de ces vis, par exemple par l'incorporation d'un double filet distal, sans pour autant s'attacher au temps global de l'intervention chirurgicale tout en conservant d'excellentes propriétés de compression et de tenue mécanique.

On connaît également les vis classiques chirurgicales auto-perforantes et auto-taraudantes, la partie taraudante de ces vis connues étant néanmoins limitée à la partie distale de la vis. En effet, les vis connues de l'art antérieur possèdent une tête de vis non filetée que l'on doit précisément enfouir également dans l'os en réalisant un chambrage préalable adapté à l'aide d'un outil spécifique. Ces vis nécessitent donc l'utilisation d'un outil supplémentaire, ce qui implique une opération additionnelle conduisant à un nombre de manipulations accrues, à un risque supplémentaire d'incidents ou de mise en place défectueuse, ce qui au total se traduit par un allongement de l'intervention chirurgicale.

La présente invention vise en conséquence à porter remède aux différents inconvénients énumérés précédemment et à proposer une nouvelle vis d'ostéosynthèse et de compression destinée à la coaptation de petits fragments d'os, qui soit auto-perforante et auto-taraudante de manière à simplifier et maîtriser au mieux sa mise en place.

Une autre objet de l'invention vise à proposer une nouvelle vis d'ostéosynthèse qui tout en étant auto-perforante et auto-taraudante, présente d'excellentes propriétés de résistance mécanique.

Une autre objet de l'invention vise à proposer une nouvelle vis d'ostéosynthèse présentant d'excellentes propriétés de taraudage tout en étant particulièrement bien équilibrée sur le plan géométrique.

Un autre objet de l'invention vise à proposer une nouvelle vis d'ostéosynthèse présentant d'excellentes propriétés de compression tout en étant particulièrement bien équilibrée sur le plan mécanique.

Les objets assignés à l'invention sont atteints à l'aide d'une vis d'ostéosynthèse et de compression destinée à la coaptation de petits fragments d'os formée par un corps longitudinal unique avec un axe longitudinal comprenant :
- une partie proximale formée par une tête de vis pourvue d'un filetage externe, ladite partie proximale étant d'un diamètre supérieur à celui du reste de la vis,
- une partie intermédiaire dépourvue de filetage,
- une partie distale pourvue d'un filetage externe,
**caractérisée en ce que :**
- la tête de vis et la partie distale comportent chacune au moins une gorge, d'une part s'étendant sensiblement longitudinalement sur toute la longueur axiale de chaque filetage, et d'autre part ménagée à travers chaque filetage de manière à former des moyens de taraudage,
- la zone terminale de la partie distale est pourvue de moyens de préparation dans les fragments d'os d'un logement destiné à recevoir les parties intermédiaire et distale.

D'autres avantages de l'invention seront explicités plus en détail à la lecture de la description qui suit, et à l'aide des dessins annexés fournis à titre purement explicatif et non limitatif, dans lesquels :

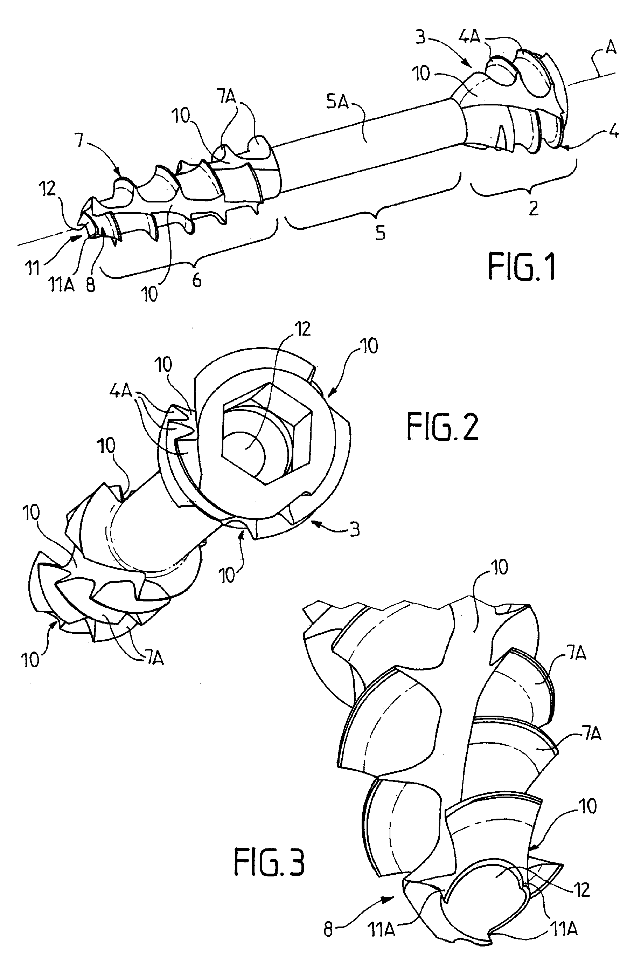

- Les figures 1 et 2 illustrent selon des vues en perspective générale, un exemple de réalisation d'une vis d'ostéosynthèse et de compression conforme à l'invention.
- La figure 3 illustre, selon une vue en perspective partielle, la conformation de la partie la plus distale de la vis conforme à l'invention.

La vis d'ostéosynthèse et de compression illustrée aux figures est destinée à assurer la solidarisation ou la coaptation de deux petits fragments d'os et en particulier, à titre d'application préférentielle, de deux phalanges du pied ou de la main qui ont été fracturées.

La vis d'ostéosynthèse et de compression conforme à l'invention est réalisée à partir d'un matériau métallique biocompatible et est formée par un corps longitudinal unique présentant un axe longitudinal A formant un axe de révolution.

Telle qu'illustrée aux figures, la vis d'ostéosynthèse comprend une partie proximale 2 formée par une tête de vis 3 pourvue d'un filetage externe 4 comprenant une série de filets hélicoïdaux 4A, ladite partie proximale 2 étant d'un diamètre D et supérieure au reste de la vis.

La vis d'ostéosynthèse conforme à l'invention comporte ensuite une partie intermédiaire 5 dépourvue de filetage, se présentant avantageusement sous la forme d'une partie sensiblement cylindrique 5A, lisse, de diamètre constant.

La partie terminale de la partie intermédiaire 5 se prolonge par la partie distale 6 de la vis d'ostéosynthèse, ladite partie distale étant pourvue d'un filetage externe 7 s'étendant de manière hélicoïdale, par l'intermédiaire de ses filets 7A jusqu'à la zone terminale 8 de ladite partie distale 6.

Selon l'invention, la tête de vis 3 et la partie distale 6 comportent chacune au moins une gorge 10, d'une part s'étendant sensiblement longitudinalement, en considérant la direction générale donnée par l'axe longitudinal A, sur toute la longueur axiale de chaque filetage 4, 7, quelle que soit la géométrie de la gorge 10, c'est-à-dire par exemple longitudinale ou avantageusement hélicoïdale, et d'autre part ménagées à travers chaque filetage 4, 7, c'est à dire à travers la hauteur de chaque filet 4A, 7A, de manière à former des moyens de taraudage de la vis. La vis selon l'invention comporte donc au moins une paire de gorges 10 pour réaliser un taraudage pour la partie distale et la partie proximale.

Selon l'invention, la vis d'ostéosynthèse comprend également au niveau de la zone terminale 8 de la partie distale 6, des moyens de préparation 11, dans les fragments d'os, d'un logement destiné à recevoir ultérieurement les parties intermédiaire 5 et distale 6 de ladite vis.

Avantageusement, l'effet de compression est obtenu en prévoyant de réaliser la vis avec un filetage de la partie proximale 2 qui d'un pas inférieur au filetage de la partie distale 6.

Grâce aux moyens techniques ainsi mis en oeuvre, la vis d'ostéosynthèse conforme à l'invention permet non seulement une mise en compression axiale des deux fragments d'os à solidariser grâce à la présence des deux filetages externes 4 et 7, en combinaison avec la partie intermédiaire 5, dont l'aspect lisse permet le glissement relatif des deux fragments d'os l'un vers l'autre, mais encore une action auto-perforante grâce aux moyens de préparation 11 et surtout une action auto-taraudante conjointe et simultanée au niveau de la tête de vis 3 et de la partie distale 6. Cette particularité de conception évite d'avoir recours à un perçage ou chambrage préalable à l'aide d'un instrument spécifique ce qui réduit la durée totale de l'intervention chirurgicale tout en réduisant les risques de mise en place défectueuse en raison de l'existence d'une seule et unique action et opération de mise en place et de compression.

Telle qu'illustrée aux figures, ladite au moins une gorge 10 présente aussi bien au niveau de la tête de vis 3 que de la partie distale 6, est avantageusement de forme hélicoïdale et de même orientation géométrique au niveau de la tête de vis 3 et de la partie distale 6 autour de l'axe longitudinal A. L'aspect hélicoïdal des gorges 10 peut être plus ou moins prononcé selon la taille et la conformation de la vis sans pour autant sortir du cadre de l'invention. L'orientation géométrique de l'hélice peut être qualifiée en définissant son obliquité ou orientation angulaire par rapport à l'axe longitudinal A de la vis. A titre préférentiel, l'obliquité de chaque gorge 10 sera avantageusement comprise entre 20° et 40°, et préférentiellement d'environ 25°. Avantageusement, les obliquités des gorges 10 de la tête de vis 3 et de la partie distale 4 sont égales.

Bien évidemment, à titre de variante, la ou les paires de gorges 10 peuvent être sensiblement longitudinale(s), c'est à dire s'étendre sensiblement parallèlement à l'axe longitudinal A sans pour autant sortir du cadre de l'invention.

La profondeur de chaque gorge 10 peut être constante et égale entre les gorges de la tête de vis 3 et de la partie distale 6.

Néanmoins, de manière avantageuse, la profondeur des gorges 10 est régulièrement variable du début vers la fin de chaque gorge, le début de chaque gorge 10 étant, par définition au sens de l'invention, considéré comme étant celui débutant à la partie la plus proximale de la tête de vis 3 et de la partie distale 6. Ainsi, selon l'invention, la profondeur de chaque gorge 10 croît en direction de chaque partie la plus distale de la tête de vis 3 et de la partie distale 6. Ainsi, la profondeur de chaque gorge est de plus en plus importante dans chaque filetage 10 à partir de la tête de vis 3 vers la partie distale 6, c'est à dire vers la zone terminale 8.

Cette particularité qui permet de ne pas avoir une profondeur de gorge 10 constante par rapport à l'axe longitudinal A de la vis, améliore la résistance mécanique générale de la vis, en particulier à la torsion, en réduisant sensiblement la perte de matière relativement audit axe longitudinal A.

La minimisation de la faiblesse relative mécanique de la vis selon l'invention est par ailleurs rendue également possible par le recours à des gorges 10 hélicoïdales permettant de répartir des zones de faiblesse résultant de l'enlèvement de matières causées par la gorge 10, autour de l'axe longitudinal A.

De manière particulièrement avantageuse, la section transversale des gorges 10 forme un angle aigu, dans tous les cas inférieur à 90°, afin de réduire la masse de matière enlevée à la vis ce qui diminue une nouvelle fois la fragilisation de la vis sans obérer ses propriétés d'auto-taraudage.

Dans le cas de variante de réalisation préférée au sens de l'invention, mettant en oeuvre une croissance régulière de la profondeur de chaque gorge 10 en direction de la pointe de la vis, la fraction terminale de la gorge, c'est à dire sa fraction la plus profonde, sera avantageusement ménagée à travers l'épaisseur du corps de vis, alors que la portion de début de chaque gorge 10 est simplement réalisée à travers l'épaisseur des filets 4A, 7A. On réduit ainsi les risques de fragilisation mécanique de l'ensemble de la vis.

A titre de variante préférentielle, les moyens de préparation 11 sont formés par une dent 11 A s'étendant sensiblement axialement.

Bien que dans sa forme la plus simple, la vis d'ostéosynthèse conforme à l'invention peut comporter une seule paire de gorges 10 au niveau de la tête de vis 3 et de la partie distale 6, on comprend qu'il est possible d'améliorer les propriétés d'auto-perforation et d'auto-taraudage de la vis en ménageant deux paires de gorges 10, voire de manière encore plus préférentielle, trois paires de gorges 10 régulièrement réparties angulairement autour de l'axe longitudinal A et ménagées à la fois dans les parties proximale et distale de la vis.

De manière particulièrement avantageuse, les deux gorges 10 d'une même paire, qu'elles soient longitudinales ou hélicoïdales, peuvent être ménagées dans les parties proximale 2 et distale 6 de la vis, de manière à être dans le prolongement l'une de l'autre.

Bien évidemment, à titre de variante, les deux gorges 10 d'une même paire peuvent également être ménagées dans les parties proximale 2 et distale 6 de la vis, de manière à être décalées l'une de l'autre, de telle sorte que la gorge 10 ménagée dans la partie distale 6 de la vis ne soit pas dans le prolongement de la gorge 10 ménagée dans la partie proximale 2.

Ce décalage peut être réalisé avec des gorges 10 longitudinales ou hélicoïdales.

Dans le cas de deux gorges 10, ces dernières seront avantageusement opposées diamétralement alors que dans le cas préféré de trois gorges 10, ces dernières seront disposées à 120° par rapport à l'axe principal A de la vis.

Bien évidemment, il est envisageable, suivant la taille des vis concernées, de réaliser quatre, voire plus, paires de gorges 10.

La vis d'ostéosynthèse conforme à l'invention pourrait être également, mais non exclusivement, pourvue d'un perçage central 12 et longitudinal pour former une vis cannulée tel que cela est bien connu de l'homme du métier.

Bien évidemment, dans le cas où la vis d'ostéosynthèse conforme à l'invention est pourvue de trois paires de gorges 10, les moyens de préparation 11 seront simultanément formés par un nombre apparié de trois dents 11 A disposées à la jonction entre les trois gorges 10 de la partie distale 6 et le perçage central 12.

Les moyens techniques mis en oeuvre dans la vis d'ostéosynthèse et de compression conforme à l'invention permettent ainsi au chirurgien de disposer d'une vis d'ostéosynthèse auto-perforante et auto-taraudante qui peut être mis en place de manière simplifiée, en faisant appel à un nombre limité d'outils sans pour autant obérer ses propriétés de résistance mécanique et de compression.

En effet, le chirurgien, après avoir positionné relativement les deux fragments d'os à solidariser, peut tout simplement mettre en place la vis d'ostéosynthèse conforme à l'invention puis débuter directement le vissage puisque la vis est à la fois auto-perforante et surtout auto-taraudante sur toute sa longueur.

Il n'est ainsi plus nécessaire de faire appel à un outil annexe, préalablement à la mise en place de la vis pour assurer la préparation et le taraudage non seulement de la partie distale et intermédiaire de la vis mais encore de la tête de vis 3.

La présente invention concerne donc également une nouvelle méthode d'intervention chirurgicale dans laquelle la vis d'ostéosynthèse assure elle-même, grâce à sa géométrie, et par simple vissage, à l'aide d'un tournevis, la préparation de son logement et de son taraudage sans que l'on ait besoin de préperçage.

## Revendications

1. Vis d'ostéosynthèse et de compression destinée à la coaptation de petits fragments d'os formée par un corps longitudinal unique avec un axe longitudinal (A) comprenant :
- une partie proximale (2) formée par une tête de vis (3) pourvue d'un filetage externe (4), ladite partie proximale (2) étant d'un diamètre supérieur à celui du reste de la vis,
- une partie intermédiaire (5) dépourvue de filetage,
- une partie distale (6) pourvue d'un filetage externe (7),
- au moins une paire de gorges (10) respectivement présentes au niveau de la partie distale (6) et de la partie proximale (2) pour réaliser un taraudage pour la partie distale (6) et la partie proximale (2),
la gorge (10) présente au niveau de la partie distale (6) étant de forme hélicoïdale et s'étendant sensiblement sur toute la longueur axiale du filetage (7) de ladite partie distale, la zone terminale (8) de la partie distale (6) étant pourvue de moyens de préparation (11) dans les fragments d'os d'un logement destiné à recevoir les parties intermédiaire (5) et distale (6), lesdits moyens de préparation comprenant au moins une dent (11A) s'étendant sensiblement axialement.

2. Vis selon la revendication 1 **caractérisée en ce que** l'obliquité de chaque gorge hélicoïdale (10) est comprise entre 20° et 40°, préférentiellement d'environ 25°.

3. Vis selon l'une des revendications 1 et 2 **caractérisée en ce que** la profondeur des gorges (10) est constante.

4. Vis selon l'une des revendications 1 et 2 **caractérisée en ce que** la profondeur des gorges (10) est régulièrement variable du début vers la fin de chaque gorge (10).

5. Vis selon la revendication 4 **caractérisée en ce que** la profondeur de chaque gorge (10) croît en direction de la zone terminale (8) de vis.

6. Vis selon la revendication 5 **caractérisée en ce que** la portion finale de chaque gorge (10) est réalisée jusqu'à travers l'épaisseur du corps de vis.

7. Vis selon l'une des revendications 1 à 6 **caractérisée en ce qu'**elle est pourvue d'un perçage central et longitudinal (12) pour former une vis canulée.

8. Vis selon la revendication 7 **caractérisée en ce que** la jonction entre la gorge (10) et le perçage central (12) comporte ladite au moins une dent (11A) formant les moyens de perforations (11).

9. Vis selon l'une des revendications 1 à 8 **caractérisée en ce que** le diamètre de la partie proximale (2) croît en direction de l'extrémité proximale de la vis.

10. Vis selon l'une des revendications 1 à 9 **caractérisée en ce que** la partie proximale (2) s'étend axialement selon une longueur qui est inférieure à la longueur selon laquelle la partie distale (6) s'étend axialement.

11. Vis selon la revendication 10 **caractérisée en ce que** la gorge (10) présente au niveau de la partie proximale (2) est longitudinale.

12. Vis selon la revendication 10 **caractérisée en ce que** la gorge (10) présente au niveau de la partie proximale (2) est hélicoïdale.

13. Vis selon l'une des revendications 1 à 12 **caractérisée en ce que** la partie intermédiaire (5) se présente sous la forme d'une partie sensiblement cylindrique (5A), lisse, de diamètre constant.

14. Vis selon l'une des revendications 1 à 13 **caractérisée en ce que** la partie proximale (2) est pourvu d'un filetage dont le pas est inférieur au filetage de la partie distale (6).

15. Vis selon l'une des revendications 1 à 14 **caractérisée en ce qu'**elle est conçue pour la solidarisation de fragments de phalanges d'orteils ou de doigts.
